# EUROPÄISCHE PATENTANMELDUNG

(11) **EP 3 243 485 A1**
(43) Veröffentlichungstag der Anmeldung: **15.11.2017**
(21) Anmeldenummer: 16169259.5
(22) Anmeldetag: 11.05.2016
(51) Int. Cl.: A61F 2/24

(54) **HALTEVORRICHTUNG FÜR EINEN NAHTRING**

(71) Anmelder: Berlin Heart GmbH, 12247 Berlin (DE)
(72) Erfinder: KRAMBECK, Helge, 12163 Berlin (DE); LAUTERBACH, Gerhard, 12679 Berlin (DE); von WINTERFELD, Felix, 12165 Berlin (DE)
(74) Vertreter: Pfenning, Meinig & Partner mbB

(57) **Zusammenfassung**

Die Erfindung bezieht sich auf eine Haltevorrichtung für einen Nahtring (2, 2') mit einer Aufnahme (7), in der ein Nahtring (2, 2') lösbar fixierbar, insbesondere einklemmbar ist, sowie mit einem mit der Aufnahme verbundenen Handgriff (8), der eine Betätigungseinrichtung (8a, 8b) zur Fixierung und/oder Lösung der Verbindung zwischen der Aufnahme und dem Nahtring aufweist. Mittels der Haltevorrichtung ist ein Nahtring in der Aufnahme fixierbar, so dass ein rohrförmiges Teil/Objekt (5) in den Nahtring einschiebbar ist, ohne Kräfte auf das Gewebe auszuüben, mit dem der Nahtring verbunden ist.

## Beschreibung

Die Erfindung liegt auf dem Gebiet der Mechanik, der Feinmechanik und der Medizintechnik. Mit besonderem Vorteil ist die Erfindung auf dem Gebiet der Chirurgie einsetzbar.

In der Chirurgie und Implantationstechnik stellt sich oft die Aufgabe, eine Kanüle, ein Rohr, einen Rohrstutzen oder eine Blutpumpe an einem Blutgefäß oder unmittelbar am Herzen des Patienten zu befestigen. Dabei soll die Befestigung für das Gewebe schonend ausgeführt, zuverlässig und möglichst dicht sein. Üblicherweise werden für diesen Zweck sogenannte Nahtringe verwendet, die einerseits am Gewebe des Gefäßes oder des Herzens festnähbar sind und andererseits einen Anschluss für eine Kanüle, ein Rohr oder einen Rohrstutzen bieten. Zu diesem Zweck bietet ein derartiger Nahtring einen vernähbaren Teil, der beispielsweise aus einem Gewebe gebildet sein kann, und einen festen Ring zum Einstecken eines Gegenstücks. Nach dem Vernähen des Nahtrings mit dem Gewebe wird üblicherweise mit einem chirurgischen Werkzeug (Ringmesser oder Stanzwerkzeug) das Myocard innerhalb des Nahtrings ausgeschnitten, um Zugang zu dem entsprechenden Ventrikel oder auch Vorhof zu schaffen. In diesen Zugang wird dann eine Kanüle oder ein Rohr eingeführt oder eingesteckt. Das Rohr oder die Kanüle kann unmittelbar Teil einer Blutpumpe sein.

Üblicherweise ist das Teil, das in den Nahtring eingesteckt wird, passgenau ausgeführt, so dass eine möglichst feste und dichte Steckverbindung geschaffen wird. Dadurch entstehen jedoch bei der Verbindung zwischen dem Nahtring und dem einzusteckenden Teil während des Herstellens der Verbindung oder des Einsteckens Kräfte, die durch den Nahtring auf das Gewebe übertragen werden. Beispielsweise kann zwischen den zu verbindenden Teilen eine Dichtung in Form eines elastomeren O-Rings vorgesehen sein, die die Einsteckkräfte erhöht. Zudem kann ein Verriegelungselement vorgesehen sein, dass nach dem Herstellen der Verbindung eingesteckt oder eingeschoben wird, um ein nachfolgendes Abziehen des eingesteckten Teils zu verhindern.

Insbesondere bei einem geschwächten Herzen kann der Herzmuskei dünnwandig und labil sein. Dadurch besteht die Gefahr, dass das Herz an der Konnektierungsstelle verformt wird und dass das Gewebe möglicherweise einreißt, was zu einem sehr hohen Blutverlust des Patienten und zu weiterem Behandlungsbedarf führen kann.

Der vorliegenden Erfindung liegt daher die Aufgabe zugrunde, eine Haltevorrichtung für einen Nahtring zu schaffen, die ein schonendes Herstellen der Verbindung zwischen dem Nahtring und einem in diesen einzusteckenden Teil, insbesondere einem Rohr, Rohrstutzen oder einer Kanüle, erlaubt.

Die Aufgabe wird mit den Merkmalen gemäß Patentanspruch 1 durch eine Haltevorrichtung gelöst. Die Patentansprüche 2 bis 12 stellen besondere Ausführungsformen dar. Der Patentanspruch 13 bezieht sich auf ein Verfahren zur Herstellung einer Steckverbindung zwischen einem Nahtring und einem rohrförmigen Teil.

Demgemäß bezieht sich die Erfindung auf eine Haltevorrichtung für einen Nahtring mit einer Aufnahme, in der ein Nahtring lösbar fixierbar, insbesondere einklemmbar ist, sowie mit einem mit der Aufnahme verbundenen Handgriff, der eine Betätigungseinrichtung zur Fixierung und/oder Lösung der Verbindung zwischen der Aufnahme und dem Nahtring aufweist.

Durch die Betätigungseinrichtung kann der Nahtring fest in der Aufnahme der Haltevorrichtung fixiert werden. Auf diese Weise kann der Nahtring nachfolgend während des Einsteckens beispielsweise eines rohrförmigen Teils mittels der Haltevorrichtung bequem gehalten werden, so dass Kräfte, die beim Einstecken auf den Nahtring wirken, mittels der Haltevorrichtung abgefangen werden können und demgemäß nicht auf das Gewebe wirken, mit dem der Nahtring vernäht ist.

Nach dem Einstecken des Objekts in den Nahtring kann durch Betätigung der Betätigungseinrichtung und des Handgriffs die Verbindung zwischen der Haltevorrichtung und dem Nahtring wieder gelöst und der Nahtring auf diese Weise losgelassen werden.

Eine mögliche Ausgestaltung der Haltevorrichtung kann vorsehen, dass die Aufnahme eine Klemmvorrichtung mit gegeneinander beweglichen Klemmelementen aufweist.

Dabei kann vorgesehen sein, dass die Aufnahme nach Art einer Zange mit zwei Klemmbacken ausgebildet ist.

Um den Nahtring in eine definierte Position bringen zu können, kann vorgesehen sein, dass ein Nahtring in einer definierten Position relativ zur Aufnahme fixierbar, insbesondere einklemmbar ist. Einerseits ist damit der Nahtring mittels der Haltevorrichtung leicht in eine definierte Position bringbar, andererseits ist auch die Position des Nahtrings relativ zur Haltevorrichtung zuverlässig und reproduzierbar festgelegt.

Die Haltevorrichtung kann zu diesem Zweck Ausnehmungen an den Klemmbacken aufweisen, in die der Nahtring hineinpasst. Es können auch besondere Ausformungen der Klemmbacken vorgesehen sein, die mit entsprechend komplementär ausgeformten Vorsprüngen oder Ausnehmungen des Nahtrings zusammenpassen, so dass derart durch eine mechanische Kodierung die Relativposition zwischen dem Nahtring und der Haltevorrichtung eindeutig und reproduzierbar festgelegt ist.

Es kann weiter vorgesehen sein, dass der Handgriff zwei Hebelarme aufweist, die mit je einer Klemmbacke verbunden und gegeneinander schwenkbar sind. Auf diese Weise sind die Hebelarme leicht mit einer Hand eines Operateurs oder einer Hilfskraft derart betätigbar, dass der Nahtring in der Haltevorrichtung festgeklemmt und festgehalten ist.

Es kann weiterhin vorgesehen sein, dass jeder der Hebelarme mit je einer Welle verbunden ist, welche bei einer Schwenkbewegung der Hebelarme gegeneinander gedreht werden, wobei jede der Wellen mit einer der Klemmbacken verbunden ist.

Zudem kann dabei vorgesehen sein, dass eine gemeinsame Schwenkebene der Hebelarme gegenüber einer gemeinsamen Schwenkebene der Klemmbacken parallel versetzt ist. Durch den Versatz der Schwenkebenen der Hebelarme einerseits und der Klemmbacken andererseits kann ein Nahtring auch an schwierig zugänglichen Stellen ohne größere Schwierigkeiten gehalten werden, ohne dass die Hebelarme des Handgriffs an einer Stelle nahe dem Nahtring betätigt werden müssen. Der Handgriff mit seinen Hebelarmen kann vielmehr gegenüber den Klemmbacken senkrecht zur Schwenkebene der Klemmbacken versetzt sein, beispielsweise um einige Zentimeter bis zu einigen zehn Zentimetern. Damit wird während der Operation Platz in der Nähe des Nahtrings frei, bzw. die Öffnung des Patientenkörpers kann reduziert werden.

Es kann zudem vorgesehen sein, dass die Haltevorrichtung eine Führung für ein in den Nahtring einzuführendes Objekt aufweist, die eine Bewegung des Objekts in Richtung auf die Position führt, die ein in der Haltevorrichtung fixierter Nahtring einnimmt. Durch die Führung wird das Einführen eines Objekts, insbesondere eines rohrförmigen Teils, in die Öffnung des Nahtrings vereinfacht. Einerseits wird es erleichtert, mit dem einzuführenden Objekt die Öffnung des Nahtrings zu treffen, andererseits können die auftretenden Kräfte und Bewegungen und damit auch der negative Einfluss auf das Gewebe, mit dem der Nahtring verbunden ist, begrenzt werden.

Es kann außerdem vorgesehen sein, dass die Haltevorrichtung eine Konnektierungsvorrichtung zur Herstellung einer Verbindung zwischen einem Objekt und einem Nahtring aufweist, die einerseits an einem Teil der Haltevorrichtung abgestützt und andererseits dazu eingerichtet ist, das Objekt in Richtung zu einer Position zu verschieben, die ein in der Haltevorrichtung fixierter Nahtring einnimmt.

Wenn beim Einführen des Objekts / rohrförmigen Teils in die Öffnung des Nahtrings die Konnektierungsvorrichtung einerseits mit der Haltevorrichtung verbunden ist und sich andererseits an dem einzuführenden Objekt abstützt, kann eine Kraft auf das Objekt ausgeübt werden, die unmittelbar in der Konnektierungsvorrichtung oder an der Haltevorrichtung abgefangen werden kann, so dass der Nahtring dort abgestützt ist und keine Kraft auf das Gewebe ausgeübt wird, mit dem der Nahtring verbunden ist.

Dabei kann vorgesehen sein, dass die Konnektierungsvorrichtung durch einen an der Haltevorrichtung gelagerten Hebel antreibbar ist. Mittels einer Hebelvorrichtung kann die Kraft auf das Objekt über- oder untersetzt werden, so dass auch höhere Kräfte, die zum Einschieben in den Nahtring und zum Überwinden der Reibungskräfte notwendig sind, in einfacher und ungefährlicher Weise erzeugt werden können. Gleichzeitig kann mit der Hebelmechanik der Weg, den das Objekt beim Eindrücken in den Nahtring zurücklegt, sicher begrenzt oder die Endposition des Objekts im Nahtring definiert werden.

Die Haltevorrichtung kann hierzu derart eingerichtet sein, dass ein Teil der Konnektierungsvorrichtung in einer ersten Position derart positionierbar ist, dass das freie Anordnen eines Objekts vor dem Nahtring ermöglicht ist und dass der Teil in eine zweite Position hinter das Objekt oder hinter einen Vorsprung des Objekts schwenkbar ist, derart, dass mittels des Hebels ein Einschieben des Objekts in den Nahtring ermöglicht ist.

Sinnvollerweise ist die Haltevorrichtung weiter so eingerichtet, dass die Antriebsbewegung des Hebels in einer dritten Position derart begrenzt ist, dass mit dem Ende der Antriebsbewegung des Hebels ein zu konnektierendes Objekt im Nahtring seine Endposition erreicht. Nachdem das Objekt im Nahtring seine Endposition erreicht hat, kann die Haltevorrichtung gelöst und vom Nahtring entfernt werden. Es können zur Erleichterung des Einschiebens des Objekts in den Nahtring auch Markierungen vorgesehen sein, die das Erreichen einer Endposition der Konnektierungsvorrichtung und des einzuschiebenden Objekts anzeigen. Hierzu kann beispielsweise ein Pressstempel für eine Pressplatte der Konnektierungsvorrichtung in seiner Endposition mit dem Rand einer Führungsöffnung, in der er geführt ist, bündig abschließen.

Die Erfindung bezieht sich außer auf eine Haltevorrichtung der oben beschriebenen und erläuterten Art auch auf ein Verfahren zur Herstellung einer Verbindung zwischen einem Nahtring und einem Objekt in Form eines in diesen einsteckbaren rohrförmigen Teils, wobei der Nahtring mit einer Haltevorrichtung nach einem der Ansprüche 1 bis 12 gehalten wird und wobei das rohrförmige Teil in die Haltevorrichtung eingeführt und durch Betätigung eines Hebels einer Konnektierungsvorrichtung in den Nahtring eingeschoben wird.

Im Folgenden wird die Erfindung anhand von Ausführungsbeispielen in Figuren einer Zeichnung gezeigt und nachfolgend erläutert. Dabei zeigt
- Fig. 1: schematisch einen Teil der Oberfläche eines Herzmuskels mit einem aufgesetzten Nahtring in perspektivischer Ansicht,
- Fig. 2: einen Nahtring mit einem rohrförmigen, in diesen einzuschiebenden Objekt,
- Fig. 3: eine Haltevorrichtung in perspektivischer Ansicht,
- Fig. 4: eine Haltevorrichtung in einer Seitenansicht mit einer Konnektierungsvorrichtung in einer ersten Position,
- Fig. 5: eine Haltevorrichtung in einer Seitenansicht mit einer Konnektierungsvorrichtung in einer zweiten Position,
- Fig. 6: eine Haltevorrichtung mit einer Konnektierungsvorrichtung in einer dritten Position,
- Fig. 7: Klemmbacken der Haltevorrichtung im Zusammenwirken mit einem Nahtring sowie
- Fig. 8: eine weitere Ausführung von Klemmbacken im Zusammenwirken mit einem Nahtring.

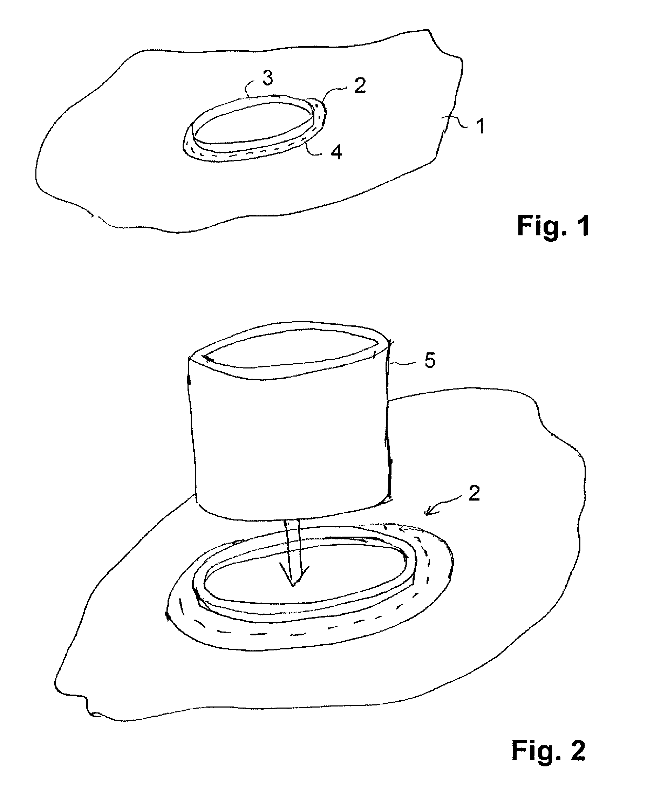

Figur 1 zeigt in einer perspektivischen Darstellung einen Teil der Oberfläche eines Patientenherzens 1, auf den ein Nahtring 2 aufgesetzt ist. Ein solcher Nahtring besteht aus einem festen, oft metallischen Ringteil 3, der mit einem vernähbaren Flanschteil 4 fest verbunden ist. Der vernähbare Flanschteil kann gewebeartig oder folienartig ausgeführt sein, so dass es mit dem Gewebe, an dem der Nahtring befestigt werden soll, bei der Implantation durch einen Chirurgen vernähbar ist.

Figur 2 zeigt den bereits in Figur 1 dargestellten Nahtring 2 im Zusammenwirken mit einem rohrförmigen Teil oder Objekt 5, das in den Nahtring 2 einzusetzen ist.

Üblicherweise wird nach dem Vernähen des Nahtrings 2 aus dem Gewebe, also beispielsweise der Herzwand, auf die der Nahtring aufgesetzt ist, innerhalb des Nahtrings ein kreisförmiger Abschnitt ausgeschnitten oder ausgestochen, um eine Öffnung in der Herzwand zu schaffen. An diese Öffnung soll ein rohrförmiges Element/Objekt 5 angekoppelt werden. Zu diesem Zweck wird das rohrförmige Element/Objekt 5 in den Nahtring eingeschoben. Die Maße des Nahtrings und des rohrförmigen Teils/Objekts 5 sind derart aufeinander abgestimmt, dass das rohrförmige Teil/Objekt 5 in dem Nahtring fest und dicht sitzt. Zu diesem Zweck können entweder in dem rohrförmigen Teil/Objekt 5 oder in dem Nahtring 2 auch Dichtelemente vorgesehen sein.

Eine derart dichte Verbindung führt dazu, dass beim Einschieben des rohrförmigen Teils/Objekts 5 in den Nahtring 2 nicht unerhebliche Kräfte auftreten. Diese können wenigstens teilweise dadurch abgefangen werden, dass ein Chirurg während der Operation den Nahtring 2 beim Einschieben des rohrförmigen Teils/Objekts 5 festhält. Ein solches Vorgehen ist aber nicht immer und in allen Situationen befriedigend. Durch die erfindungsgemäße Haltevorrichtung wird bezüglich dieses Problems Abhilfe geschaffen.

Das rohrförmige Teil/Objekt 5 ist hier nur beispielhaft dargestellt und kann einen Rohrstutzen darstellen, an dem eine nicht näher gezeigte Pumpe oder eine Pumpe selbst mit einem an ihr befestigten Rohrstutzen oder eine steife Kanüle oder ein Teil einer Kanüle befestigt ist. Auch andere Objekte, die in einen Nahtring 2 eingeschoben werden müssen, sind anstelle des rohrförmigen Teils 5 denkbar.

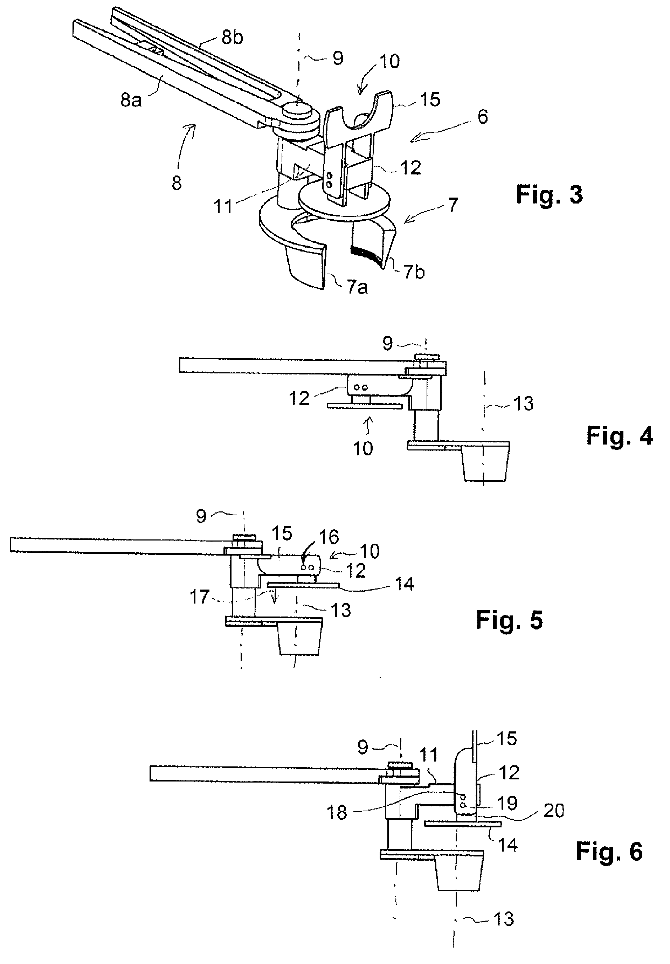

Figur 3 zeigt eine Haltevorrichtung 6 in perspektivischer Darstellung. Es ist eine Aufnahme 7 mit zwei Klemmbacken 7a, 7b gezeigt, zwischen denen ein Nahtring 2 einklemmbar ist.

Die beiden Klemmbacken 7a, 7b sind um eine gemeinsame Achse 9 gegeneinander schwenkbar, so dass ein Nahtring zwischen ihnen zangenartig einklemmbar ist. Die beiden Klemmbacken 7a, 7b sind mit zwei koaxialen, ineinander beweglichen Wellen verbunden, die entlang der Achse 9 in der Haltevorrichtung verlaufen.

Die einzelnen Wellen sind mit den beiden Hebeln 8a, 8b des Handgriffs 8 verbunden, derart, dass ein Zusammendrücken des Handgriffs 8 gleichzeitig ein Zusammendrücken der beiden Klemmbacken 7a, 7b bewirkt. Die Schwenkebenen der Hebel 8a, 8b einerseits und der Klemmbacken 7a, 7b andererseits sind gegeneinander entlang der Achse 9 versetzt. Dadurch ist die Haltevorrichtung auch an unzugänglichen Stellen leicht so einsetzbar, dass ein Nahtring einerseits durch die Klemmbacken 7a, 7b gegriffen werden kann, andererseits jedoch der Handgriff 8 an einer hiervon entfernten Stelle leicht zugänglich und betätigbar ist.

Die Haltevorrichtung 6 weist außer der Betätigungseinrichtung / dem Handgriff 8 und der Aufnahme 7 eine Konnektierungsvorrichtung 10 auf. Diese ist um die Achse 9 der Haltevorrichtung drehbar gelagert und weist einen Dreharm 11 auf, der eine Pressvorrichtung 12 trägt. Der Dreharm ist in Axialrichtung der Achse 9 festgelegt und lässt lediglich eine Drehung der Pressvorrichtung 12 um die Achse 9 zu. Mittels des Dreharms 11 ist die Pressvorrichtung an den übrigen Teilen der Haltevorrichtung abgestützt.

Figur 4 zeigt eine erste Position der Konnektierungsvorrichtung 10, in der der Dreharm 11 und die Pressvorrichtung 12 von den Klemmbacken 7a, 7b weggeschwenkt sind. In Figur 4 ist eine Achse 13 eingezeichnet, die die Mittelachse eines von den Klemmbacken 7a, 7b zu greifenden Objekts darstellt, also im Anwendungsfall die Mittelachse eines Nahtrings, der von den Klemmbacken 7a, 7b fixiert ist. Die erste Position der Konnektierungsvorrichtung ist derart gewählt, dass die Achse 13 frei ist, so dass der Raum für das Einführen eines rohrförmigen Teils/Objekts 5 frei ist.

Figur 5 zeigt die Konnektierungsvorrichtung 10 in einer zweiten Position, in der der Dreharm 11 in Richtung der Achse 13 geschwenkt ist. Nun liegen die Konnektierungsvorrichtung 10 und die Pressvorrichtung 12 oberhalb des in den Nahtring einzupressenden rohrförmigen Teils 5. Die Pressvorrichtung 12 weist eine Pressplatte 14, einen Betätigungshebel 15 und einen Hebelmechanismus 16 auf, der ein Schwenken des Betätigungshebels 15 in eine Abwärtsbewegung der Pressplatte 14 in Richtung des Pfeils 17 umsetzt.

Der Hebelmechanismus weist beispielsweise eine Welle 18 auf, mittels deren der Betätigungshebel 15 an dem Dreharm 11 gelagert ist, sowie einen Betätigungssplint 19, der in einer Kulisse eines Pressstempels 20, an dem die Pressplatte 14 befestigt ist, beweglich ist. Wird der Betätigungshebel 15 nach oben in die vertikale Position bis zu einem nicht dargestellten Anschlag geschwenkt, so betätigt der Splint 19 den Pressstempel 20 derart, dass dieser nach unten in Richtung des Pfeils 17 bis in seine Endstellung angetrieben wird. Der Pressstempel 20 ist dabei in einer Führung innerhalb des Dreharms 11 derart geführt, dass er seitlich von der Achse 13 nicht abweichen kann, sondern sich lediglich in Richtung der Achse 13 bewegen kann.

Damit wird, wenn der Betätigungshebel 15 in die in den Figuren 3 und 6 dargestellte Position bewegt wird, die Konnektierungsvorrichtung in eine dritte Position gebracht, in der das in den Nahtring einzuschiebende Teil/Objekt genau so weit vorgeschoben ist, dass die Verbindung zu dem Nahtring zuverlässig hergestellt ist. Die bei dieser Einschubbewegung wirkenden Kräfte wirken dabei ausschließlich zwischen der Aufnahme 7 und der Konnektierungsvorrichtung innerhalb der Haltevorrichtung, so dass auf das Gewebe, mit dem der Nahtring 2 verbunden ist, im Idealfall gar keine Kräfte wirken.

Die Länge der Vorschubbewegung des Pressstempels 20 der Konnektierungsvorrichtung ist auf die Größe des in den Nahtring 2 einzuschiebenden rohrförmigen Teils/Objekts 5 eingerichtet.

Um für das Funktionieren der Haltevorrichtung den Nahtring in eine definierte Position relativ zu der Haltevorrichtung zu bringen, ist es vorgesehen, dass der Nahtring in der Aufnahme 7 in einer definierten Position eingeklemmt wird.

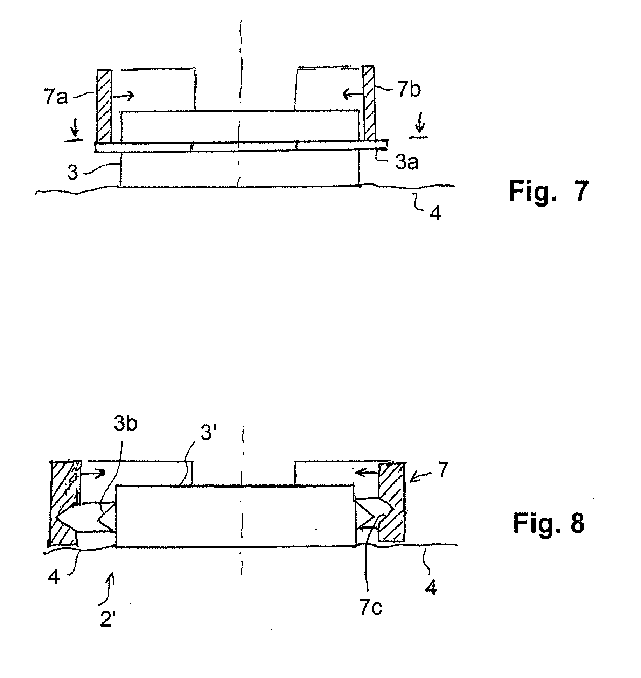

Figur 7 zeigt hierzu beispielhaft die Klemmbacken 7a, 7b, die auf einen Flansch 3a des Ringteils 3 des Nahtrings 2 aufgesetzt werden, bevor der Nahtring eingeklemmt wird. Damit ist eine Relativposition zwischen der Haltevorrichtung und dem Nahtring definiert.

In Figur 8 ist in einem weiteren Ausführungsbeispiel ein Nahtring 2' mit einem Ringteil 3' gezeigt, der einen umlaufenden, im Querschnitt dreieckigen Steg 3b aufweist, der in eine komplementäre Nut 7c der Klemmbacken der Aufnahme 7 eingreift, wenn die Klemmbacken sich um den Nahtring 2' schließen. Die Kontur des umlaufenden Steges 3b ist so gewählt, dass dieser sich in der Nut 7c zentriert und somit die Relativposition zwischen der Haltevorrichtung und dem Nahtring 2' definiert ist.

## Patentansprüche

1. Haltevorrichtung für einen Nahtring (2, 2') mit einer Aufnahme (7), in der ein Nahtring (2, 2') lösbar fixierbar, insbesondere einklemmbar ist, sowie mit einem mit der Aufnahme verbundenen Handgriff (8), der eine Betätigungseinrichtung (8a, 8b) zur Fixierung und/oder Lösung der Verbindung zwischen der Aufnahme und dem Nahtring aufweist.

2. Haltevorrichtung nach Anspruch 1, **dadurch gekennzeichnet, dass** die Aufnahme (7) eine Klemmvorrichtung mit gegeneinander beweglichen Klemmelementen (7a, 7b) aufweist.

3. Haltevorrichtung nach Anspruch 1 oder 2, **dadurch gekennzeichnet, dass** die Aufnahme (7) nach Art einer Zange mit zwei Klemmbacken (7a, 7b) ausgebildet ist.

4. Haltevorrichtung nach Anspruch 1 oder einem der folgenden, **dadurch gekennzeichnet, dass** die Aufnahme (7) derart geformt ist, dass ein Nahtring (2, 2') in einer definierten Position relativ zur Aufnahme (7) fixierbar, insbesondere einklemmbar ist.

5. Haltevorrichtung nach Anspruch 1 oder einem der folgenden, **dadurch gekennzeichnet, dass** der Handgriff (8) zwei Hebelarme (8a, 8b) aufweist, die mit je einer Klemmbacke (7a, 7b) verbunden und gegeneinander schwenkbar sind.

6. Haltevorrichtung nach Anspruch 5, **dadurch gekennzeichnet, dass** jeder der Hebelarme (8a, 8b) mit je einer Welle verbunden ist, welche bei einer Schwenkbewegung der Hebelarme gegeneinander gedreht werden, wobei jede der Wellen mit einer der Klemmbacken (7a, 7b) verbunden ist.

7. Haltevorrichtung nach Anspruch 6, **dadurch gekennzeichnet, dass** eine gemeinsame Schwenkebene der Hebelarme (8a, 8b) gegenüber einer gemeinsamen Schwenkebene der Klemmbacken (7a, 7b) parallel versetzt ist.

8. Haltevorrichtung nach Anspruch 1 oder einem der folgenden, **dadurch gekennzeichnet, dass** sie eine Führung für ein in den Nahtring einzuführendes Objekt (5) aufweist, die eine Bewegung des Objekts in Richtung (17) auf die Position führt, die ein in der Haltevorrichtung fixierter Nahtring (2, 2') einnimmt.

9. Haltevorrichtung nach Anspruch 1 oder einem der folgenden, **dadurch gekennzeichnet, dass** sie eine Konnektierungsvorrichtung (10) zur Herstellung einer Verbindung zwischen einem Objekt (5) und einem Nahtring (2, 2') aufweist, die einerseits an einem Teil der Haltevorrichtung abgestützt und andererseits dazu eingerichtet ist, das Objekt in Richtung (17) zu einer Position zu verschieben, die ein in der Haltevorrichtung fixierter Nahtring (2, 2') einnimmt.

10. Haltevorrichtung nach Anspruch 9, **dadurch gekennzeichnet, dass** die Konnektierungsvorrichtung (10) durch einen an der Haltevorrichtung gelagerten Hebel (15) antreibbar ist.

11. Haltevorrichtung nach Anspruch 10, **gekennzeichnet dadurch, dass** ein Teil (11, 14, 20) der Konnektierungsvorrichtung (10) in einer ersten Position derart positionierbar ist, dass das freie Anordnen eines Objekts (5) vor dem Nahtring (2, 2') ermöglicht ist und dass der Teil (11, 14, 20) in eine zweite Position hinter das Objekt oder hinter einen Vorsprung des Objekts schwenkbar ist, derart, dass mittels des Hebels ein Einschieben des Objekts in den Nahtring (2, 2') ermöglicht ist.

12. Haltevorrichtung nach Anspruch 10 oder 11, **dadurch gekennzeichnet, dass** die Antriebsbewegung des Hebels (15) in einer dritten Position derart begrenzt ist, dass mit dem Ende der Antriebsbewegung des Hebels ein zu konnektierendes Objekt (5) im Nahtring seine Endposition erreicht.

13. Verfahren zur Herstellung einer Verbindung zwischen einem Nahtring (2, 2') und einem Objekt mit einem in den Nahtring einsteckbaren rohrförmigen Teil (5), **dadurch gekennzeichnet, dass** der Nahtring (2, 2') mit einer Haltevorrichtung nach einem der Ansprüche 1 bis 12 gehalten wird und dass das rohrförmige Teil (5) in die Haltevorrichtung eingeführt und durch Betätigung eines Hebels (15) einer Konnektierungsvorrichtung (10) in den Nahtring eingeschoben wird.
